Europäisches Patentamt

European Patent Office

Office européen des brevets

(11) Publication number: **0 334 506**
**A2**

# EUROPEAN PATENT APPLICATION

(21) Application number: 89302260.8

(22) Date of filing: 07.03.89

(51) Int. Cl.⁴ **C12M 3/00 , C12M 1/04 , C12M 1/12**

(30) Priority: 07.03.88 JP 51538/88

(43) Date of publication of application:
27.09.89 Bulletin 89/39

(84) Designated Contracting States:
**BE DE ES FR GB IT NL**

(71) Applicant: JUNKOSHA CO. LTD.
25-25, Miyasaka 2-chome
Setagaya-ku Tokyo 156(JP)

(72) Inventor: Sakai, Mari c/o Japan Gore-Tex, Inc.
42-5 Akazutsumi, 1-chome
Setagaya-ku Tokyo 156(JP)

(74) Representative: Taylor, Derek George et al
Mathisen, Macara & Co. The Coach House
6-8 Swakeleys Road
Ickenham Uxbridge UB10 8BZ(GB)

(54) A culturing apparatus.

(57) A culturing apparatus provides a culture solution flow region (7) which is substantially surrounded by a gas-permeable membrane (8) and a supply of oxygen or other gas communicating (at 14) with the face of the membrane opposite that contacted by the culture solution whereby gas passing through the membrane is supplied to the solution. Another membranbe or part of the same membrane can communicate (at 13) with a degassing region to enable gas to pass from the solution through the membrane to the degassing region.

## FIG. 7

EP 0 334 506 A2

# A CULTURING APPARATUS

The present invention concerns the culturing of cells, microorganisms and tissues, as well as bioreactors utilising oxygen in such culturing, and especially concerns the culturing of aerobic bacteria and easily damaged animal cells.

Methods for the supply of oxygen in culturing apparatus and bioreactors used for the culturing of cells, microorganisms and tissues include absorption from the surface of the culture solution, blowing in by means of a sparger, and aeration for example by means of agitation. Furthermore, methods in which oxygen is supplied via a membrane have been described in Japanese Laid-Open Patent Application (Kokai) No. 57-159535, Japanese Laid-Open Patent Application (Kokai) No. 59-175877, Japanese Laid-Open Patent Application (Kokai) No. 60-234580 and Japanese Laid-Open Patent Application (Kokai) No. 61-100190.

Furthermore, in Bunri Gijutsu, Separation Technology, Vol. 14, No. 4 (1984), a method is introduced in which the efficiency of acetic acid fermentation is increased by fixing acetic acid bacteria (which are aerobic bacteria) to the outside of a hydrophobic hollow membrane structure, and causing air to flow through said hollow membrane structure.

In the abovementioned conventional systems involving absorption from the surface of the culture solution, there are limits to the oxygen supply capacity, and this capacity is unavoidably insufficient in cases where the culture tank is large in size. In the case of blowing in by means of a sparger of aeration by agitation, there is a high possibility of bubble or agitation damage in the case of delicate cells such as animal cells, or cells which have little resistance to a shearing force.

The methods described in the aforementioned laid-open patent applications and Bunri Gijutsu, Separation Technology, which solve the abovementioned problems, supply oxygen to a culture solution located on the outside of a semi-permeable membrane tube without generating any bubbles; this is accomplished by causing oxygen to flow through the interior of said semi-permeable membrane tube. However, since this is a calm supply state which lacks the agitating action that accompanies the rise of bubbles generated in the culture solution, a bias tends to be generated in the concentration of solute oxygen in the culture solution inside the tank, so that the concentration of solute oxygen in the culture solution near the circumference of the semi-permeable membrane is constantly high in relative terms. Accordingly, the supply efficiency is still insufficient, so that it is difficult to obtain desirable results using such a method, especially in the case of a large apparatus.

According to the present invention there is provided a culturing apparatus in which a gas is caused to contact a culture solution via a gas-permeable membrane so that said gas is supplied to said culture solution, comprising:

(a) a culture solution flow region which is substantially surrounded by the aforementioned gas-permeable membrane.

(b) a culture solution supply means which supplies culture solution to the aforementioned culture solution flow region from a supply source, and

(c) a gas supply region facing the aforementioned gas-permeable membrane which supplies gas to the flowing culture solution.

The present invention also provides a culturing apparatus in which a gas is caused to contact a culture solution via a gas-permeable membrane so that said gas is supplied to said culture solution, comprising:

(a) a culture solution flow region which is substantially surrounded by the aforementioned gas-permeable membrane,

(b) a culture solution supply means which supplies culture solution to the aforementioned culture solution flow region from a supply source, and

(c) a degassing region which degasses the flowing culture solution and a gas supply region which supplies gas to the flowing culture solution, both facing the aforementioned gas-permeable membrane.

Embodiments of the invention will now be particularly described, by way of example, with reference to the accompanying drawing in which:-

Figures 1 and 2 illustrate examples of the overall construction of apparatus according to the present invention;

Figures 3 and 4 illustrate modifications of the gas-permeable membrane tube in the gas diffusion module of the apparatus of Figures 1 and 2;

Figure 5 illustrates a double tube which constitutes another example of the aforementioned membrane tube;

Figures 6 and 7 illustrate other working configurations of the structure inside the gas-permeable membrane module.

A culture solution is supplied by a supply means, such as a pump, from a supply source, such as a culture tank, to a culture solution flow region which is virtually surrounded by a gas-permeable membrane. Specifically, this culture solution flows through the aforementioned culture solution flow region, so that the culture solution in contact with the aformentioned gas-permeable membrane is constantly renewed and effectively supplied with a gas, such as oxygen.

Such a supplying of gas via a gas-permeable membrane is an efficient method of supply which does not require the generation of bubbles. Since no bubbles are generated in the culture tank or in the vicinity of the gas-permeable membrane, damage to delicate microorganisms or cells can be avoided.

Since the aforementioned culture solution is fed into a flow region inside a gas-permeable membrane by a supply means from a supply source such as a culture tank, and is then returned to said supply source from said flow region, flow of the culture solution is also effected inside the aforementioned supply source, so that the amount of solute oxygen in the culture solution is made uniform.

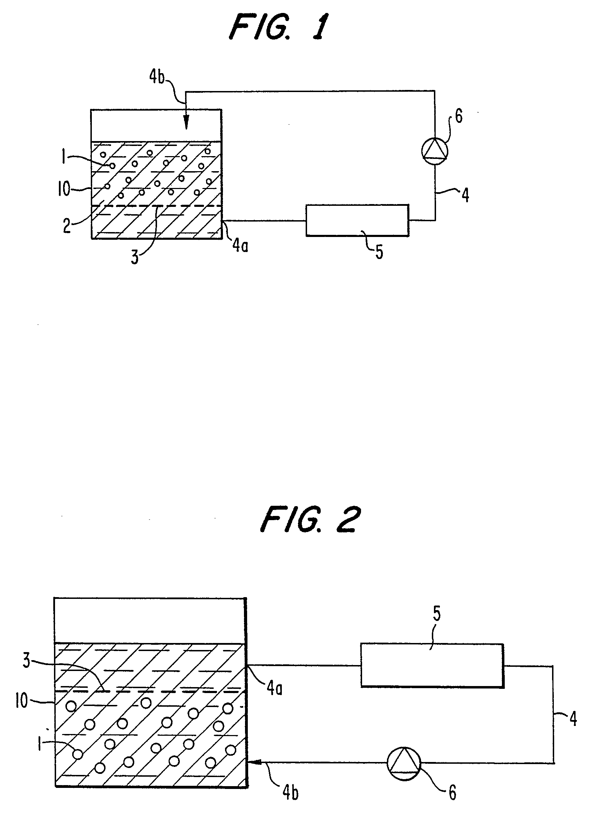

Figure 1 shows one example of the overall construction of the apparatus. The interior of a culture tank 10 is partitioned by a mesh filter 3 which does not allow the passage of the bodies 1 being cultured (e.g. cells, microorganisms or tissues). The aforementioned bodies 1 being cultured, or a support on which said cultured bodies 1 are fixed, are positioned on one side of the abovementioned partition, and a culture solution 2 is accommodated in this space. The intake port 4a of a culture solution circulation line 4 opens into the space on the other side of the abovementioned partition. A gas-permeable membrane module 5 is installed in the aforementioned culture solution circulation line 4, and the discharge port 4b of said line 4 opens into the space on the aforementioned first side of the abovementioned partition. The aforementioned culture solution 2 is supplied and circulated by a supply means 6 such as a pump.

The culture tank 10 which constitutes the culture solution supply source should naturally be maintained at a temperature which is suitable for the culturing operation in question. Meanwhile, it is desirable that the temperature conditions in the gas-permeable membrane module 5 be suitable for gas supply. Accordingly, the culture solution conducted into said module 5 may be appropriately heated or cooled. In cases where the solution temperature in the culture tank 10 and the solution temperature of the solution returned to the culture tank via the discharge port 4b are the same, or where the solution temperature at the discharge port is lower, the arrangement shown in Figure 1 is suitable. However, in cases where the solution temperature at the discharge port 4b is conversely higher than the solution temperature inside the culture tank 10, it is desirable to instal the intake port 4a in the upper part of the culture tank l0 and the discharge port 4b in the lower part of said tank 10 as shown in Figure 2, so that a relationship which is the opposite of that shown in Figure 1 is established. Specifically, as a result of either of these repective arrangements, a relationship which causes the culture solution discharged from the discharge port 4b to flow toward the intake port 4a is effectively established inside the culture tank 10. As a result, the concentration of solute gas (oxygen) in the culture solution 2 inside the tank 10 is constantly kept uniform.

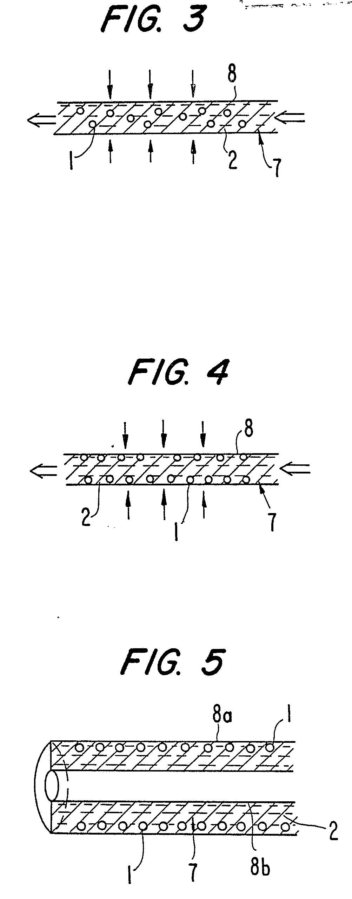

The gas-permeable membrane 8 in the aforementioned gas-permeable membrane module 5 allows gas to pass through without allowing the culture solution to pass through. A culture solution flow region 7 is formed by said gas-permeable membrane 8. In the case of the arrangements illustrated in Figures 1 and 2, only the culture solution passes through said flow region 7, and gas is supplied and diffused into said solution from the outside via the gas-permeable membrane 8. In regard to this flow region 7, however, it would also be possible to remove the aforementioned filter 3 so that both the bodies 1 being cultured and the culture solution 2 pass together through the flow region 7, as shown in Figure 3. Alternatively, it would also be possible to fix the bodies being cultured 1 in place inside the flow region 7 formed by the aforementioned gas-permeable membrane 8, and to allow only the culture solution to pass through, as shown in Figure 4. Methods which can be used for such fixing include universally known methods such as ionic bonding, covalent bonding and inclusion methods. Such fixing can easily be accomplished by such methods.

Figure 5 illustrates yet another arrangement of the aforementioned flow region 7. In this arrangement, specifically, a double tube made of the aforementioned gas-permeable membrane is used, and this double tube is arranged so that a gas such as oxygen is supplied, while carbon dioxide and/or other product gases are at the same time removed. A flow region 7 for the culture solution 2 is formed between the outside tube 8a and the inside tube 8b. Oxygen or other gas passes through the outside tube 8a and is thus supplied to the culture solution 2, while product gases from the bodies 1 being cultured, such as carbon dioxide gas, are expelled via the inside tube 8b.

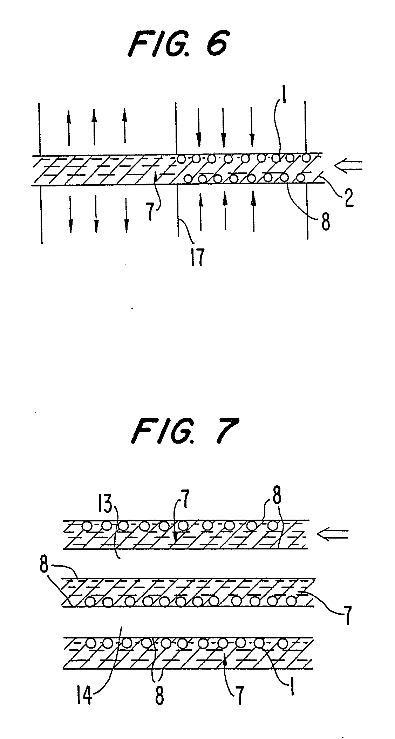

Furthermore, such supply and removal of respective gases may also be accomplished as shown in Figure 6. The space outside the gas-permeable membrane 8 can be divided by a partition 17, with the pressure on one side of the partition lowered so that product gases are removed, and the pressure on the other side of the partition appropriately raised so that gas is supplied.

3

Furthermore, it would also be possible to perform both the removal of product gases from the culture solution and the supply of a gas such as fresh oxygen, as indicated in Figure 7. Flat gas-permeable membranes 8 are installed in a multi-layer configuration, forming culture solution flow regions 7 in alternate spaces between said membranes, and forming reduced-pressure regions 13 and pressurised regions 14 between the resulting culture solution flow regions 7,7.

Furthermore, in addition to the abovementioned tube-form or flat gas-permeable membranes, the apparatus of the present invention could also employ a spiral-form or pleated module.

In the supply of gas to the culture solution via the abovementioned gas-permeable membrane 8, the fact that said gas-permeable membrane 8 is generally an extremely thin membrane makes it difficult from a practical standpoint to supply gas using an extremely large pressure differential. Furthermore, since this supply is normally accomplished efficiently, there is generally no need to employ an especially large pressure differential. However, in cases where there is a need for a large pressure differential, it is desirable that the supply operation be performed at a pressure differential of $0.15 \text{ kg/cm}^2$ or less in order to avoid damage to the bodies 1 being cultured as a result of the generation of gas bubbles in the culture solution.

The use of a water-repellent porous membrane as the aforementioned gas-permeable membrane 8 is desirable in that the properties of such a material can be utilised in order to block the passage of the culture solution, and in that the desired effective contact with the gas can be obtained using a membrane in which relatively large pores are formed. A membrane made of a fluoro-resin or silicone resin (which presents no particular danger of elution) may be employed, or a gas-separating membrane such as an oxygen enrichment membrane may be used. One example of a desirable gas-permeable membrane is a tube or membrane material with a porosity of 75 to 95 vol percent and a maximum pore size of approximately 0.02 to 3 microns, which is formed by drawing a polytetrafluoroethylene film so that siad film is made porous according to U.S. Patent 3,953,566. The gas permeability of this membrane is approximately 3 to 4.8 ml/min.cm$^2$.

As was described above, the present invention makes it possible to supply a gas to a culture solution continuously by means of an in-line system. This apparatus is able to cope effectively even with large equipment, and allows an efficient supply of oxygen or other gas to be obtained.

## Claims

1. A culturing apparatus in which a gas is caused to contact a culture solution via a gas-permeable membrane so that said gas is supplied to said culture solution, comprising:

(a) a culture solution flow region which is substantially surrounded by the aforementioned gas-permeable membrane,

(b) a culture solution supply means which supplies culture solution to the aforementioned culture solution flow region from a supply source, and

(c) a gas supply region facing the aforementioned gas-permeable membrane which supplies gas to the flowing culture solution.

2. A culturing apparatus in which a gas is caused to contact a culture solution via a gas-permeable membrane so that said gas is supplied to said culture solution, comprising:

(a) a culture solution flow region which is substantially surrounded by the aforementioned gas-permeable membrane,

(b) a culture solution supply means which supplies culture solution to the aforementioned culture solution flow region from a supply source, and

(c) a degassing region which degasses the flowing culture solution and a gas supply region which supplies gas to the flowing culture solution, both facing the aforementioned gas-permeable membrane.

# FIG. 1

# FIG. 2

## FIG. 3

## FIG. 4

## FIG. 5

## FIG. 6

## FIG. 7